# EUROPEAN PATENT SPECIFICATION

(11) **EP 3 240 476 B1**
(45) Date of publication and mention of the grant of the patent: **22.08.2018**
(21) Application number: 15813376.9
(22) Date of filing: 17.12.2015
(51) Int. Cl.: A61B 5/05

(54) **CABLING ARRANGEMENT, COIL APPARATUS AND APPARATUS FOR INFLUENCING AND/OR DETECTING MAGNETIC PARTICLES**
VERKABELUNGSANORDNUNG, SPULENVORRICHTUNG UND VORRICHTUNG ZUR BEEINFLUSSUNG UND/ODER DETEKTION MAGNETISCHER PARTIKEL
AGENCEMENT DE CÂBLES, APPAREIL DE BOBINE ET DISPOSITIF PERMETTANT D'INFLUENCER ET/OU DÉTECTER DES PARTICULES MAGNÉTIQUES

(30) Priority: 29.12.2014 EP 14200429
(43) Date of publication of application: 08.11.2017
(73) Proprietor: Koninklijke Philips N.V., 5656 AE Eindhoven (NL)
(72) Inventor: SCHMALE, Ingo, 5656 AE Eindhoven (NL); GLEICH, Bernhard, 5656 AE Eindhoven (NL); SATTEL, Timo, Frederik, 5656 AE Eindhoven (NL)
(74) Representative: Coops, Peter
(86) International application number: PCT/EP2015/080116
(87) International publication number: WO 2016/107750

(56) References cited:
- DE-B3-102012 221 838
- JP-A- H05 275 254
- US-A- 5 179 332
- US-A1- 2009 115 415
- US-A1- 2010 052 668
- US-A1- 2010 188 786
- US-A1- 2014 254 056

## Description

### FIELD OF THE INVENTION

The present invention relates to a cabling arrangement and a coil apparatus, in particular for use in a magnetic particle imaging apparatus, as well as an apparatus and method for influencing and/or detecting magnetic particles in a field of view. The present invention relates particularly to the field of Magnetic Particle Imaging.

### BACKGROUND OF THE INVENTION

Magnetic Particle Imaging (MPI) is an emerging medical imaging modality. The first versions of MPI were two-dimensional in that they produced two-dimensional images. Newer versions are three-dimensional (3D). A four-dimensional image of a non-static object can be created by combining a temporal sequence of 3D images to a movie, provided the object does not significantly change during the data acquisition for a single 3D image.

MPI is a reconstructive imaging method, like Computed Tomography (CT) or Magnetic Resonance Imaging (MRI). Accordingly, an MP image of an object's volume of interest is generated in two steps. The first step, referred to as data acquisition, is performed using an MPI scanner. The MPI scanner has means to generate a static magnetic gradient field, called the "selection field", which has a (single or more) field-free point(s) (FFP(s)) or a field-free line (FFL) at the isocenter of the scanner (in the following reference is mostly made to the field-free point, which shall however include the option of using a field-free line instead). Moreover, this FFP (or the FFL; mentioning "FFP" in the following shall generally be understood as meaning FFP or FFL) is surrounded by a first sub-zone with a low magnetic field strength, which is in turn surrounded by a second sub-zone with a higher magnetic field strength. In addition, the scanner has means to generate a time-dependent, spatially nearly homogeneous magnetic field. Actually, this field is obtained by superposing a rapidly changing field with a small amplitude, called the "drive field", and a slowly varying field with a large amplitude, called the "focus field". By adding the time-dependent drive and focus fields to the static selection field, the FFP may be moved along a predetermined FFP trajectory throughout a "volume of scanning" surrounding the isocenter. The scanner also has an arrangement of one or more, e.g. three, receive coils and can record any voltages induced in these coils. For the data acquisition, the object to be imaged is placed in the scanner such that the object's volume of interest is enclosed by the scanner's field of view, which is a subset of the volume of scanning.

The object must contain magnetic nanoparticles or other magnetic non-linear materials; if the object is an animal or a patient, a tracer containing such particles is administered to the animal or patient prior to the scan. During the data acquisition, the MPI scanner moves the FFP along a deliberately chosen trajectory that traces out / covers the volume of scanning, or at least the field of view. The magnetic nanoparticles within the object experience a changing magnetic field and respond by changing their magnetization. The changing magnetization of the nanoparticles induces a time-dependent voltage in each of the receive coils. This voltage is sampled in a receiver associated with the receive coil. The samples output by the receivers are recorded and constitute the acquired data. The parameters that control the details of the data acquisition make up the "scan protocol".

In the second step of the image generation, referred to as image reconstruction, the image is computed, or reconstructed, from the data acquired in the first step. The image is a discrete 3D array of data that represents a sampled approximation to the position-dependent concentration of the magnetic nanoparticles in the field of view. The reconstruction is generally performed by a computer, which executes a suitable computer program. Computer and computer program realize a reconstruction algorithm. The reconstruction algorithm is based on a mathematical model of the data acquisition. As with all reconstructive imaging methods, this model can be formulated as an integral operator that acts on the acquired data; the reconstruction algorithm tries to undo, to the extent possible, the action of the model.

Such an MPI apparatus and method have the advantage that they can be used to examine arbitrary examination objects - e. g. human bodies - in a non-destructive manner and with a high spatial resolution, both close to the surface and remote from the surface of the examination object. Such an apparatus and method are generally known and have been first described in DE 101 51 778 A1 and in Gleich, B. and Weizenecker, J. (2005), "Tomographic imaging using the nonlinear response of magnetic particles" in Nature, vol. 435, pp. 1214-1217, in which also the reconstruction principle is generally described. The apparatus and method for magnetic particle imaging (MPI) described in that publication take advantage of the non-linear magnetization curve of small magnetic particles.

An MPI apparatus and method are based on a new physical principle (i.e. the principle referred to as MPI) that is different from other known conventional medical imaging techniques, as for example nuclear magnetic resonance (NMR). In particular, this MPI-principle, does, in contrast to NMR, not exploit the influence of the material on the magnetic resonance characteristics of protons, but rather directly detects the magnetization of the magnetic material by exploiting the non-linearity of the magnetization characteristic curve. In particular, the MPI-technique exploits the higher harmonics of the generated magnetic signals which result from the non-linearity of the magnetization characteristic curve in the area where the magnetization changes from the non-saturated to the saturated state.

Within MPI, image information is gathered by analysing the weak non-linearity of the magnetic response of magnetic nanoparticles. This imaging can be limited in sensitivity by (potentially non-stable) harmonic background, which is generated by e.g. the non-linearity of components (e.g. capacitors), magnetic materials near the components and/or magnetic materials inside the components (e.g. magnetic iron contamination in generally non-magnetic copper). The latter is of concern in cabling, which is used at various positions in an MPI setup. In particular, high currents are conducted by cabling between and in the components of the high-current generator. The strongest magnetic field strengths in the cabling occur where the cabling is wound to serve as an inductor. Besides components of an MPI setup, this effect is largest in the drive field signal generator of the MPI apparatus.

### SUMMARY OF THE INVENTION

It is an object of the present invention to provide a cabling arrangement and a coil apparatus as well as an apparatus and a method for influencing and/or detecting magnetic particles in a field of view that reduce the non-linearity of the cabling leading to less disturbing harmonic background and thus lead to an improvement of the sensitivity of the arrangement.

In a first aspect of the present invention a cabling arrangement, in particular for use in a magnetic particle imaging apparatus, is presented comprising:
- a first AC terminal,
- a second AC terminal, wherein said first and second AC terminals are configured for coupling an AC voltage between them,
- a first internal terminal,
- a second internal terminal, wherein said first and second internal terminals are configured for coupling a DC voltage between them,
- a first subset of one or more first conductors coupled between the first internal terminal and the second AC terminal, and
- a second subset of one or more second conductors coupled between the second internal terminal and the second AC terminal,
wherein the cabling arrangement enables the superposition of the AC and DC voltages in said first and second conductors, and
wherein said first and second conductors are arranged to form a coil to generate a magnetic field in a zone of interest.

In a further aspect of the present invention a coil apparatus, in particular for use in a magnetic particle imaging apparatus, is presented comprising:
- a cabling arrangement as disclosed herein and
- a DC voltage or current source coupled, in particular via switches, between the first and second internal terminals.

In still a further aspect of the present invention an apparatus for influencing and/or detecting magnetic particles in a field of view is presented, which apparatus comprises:
- selection means comprising a selection field signal generator unit and selection field elements for generating a magnetic selection field having a pattern in space of its magnetic field strength such that a first sub-zone having a low magnetic field strength where the magnetization of the magnetic particles is not saturated and a second sub-zone having a higher magnetic field strength where the magnetization of the magnetic particles is saturated are formed in the field of view,
- drive means comprising a drive field signal generator unit and drive field coils for changing the position in space of the two sub-zones in the field of view by means of a magnetic drive field so that the magnetization of the magnetic material changes locally, and
- one or more coil apparatuses as disclosed herein forming said drive field coils, wherein the drive field signal generator unit is coupled to the AC terminals of the one or more cabling arrangements of the one or more coil apparatuses.

In another aspect of the present invention a corresponding method for influencing and/or detecting magnetic particles in a field of view is presented.

Preferred embodiments of the invention are defined in the dependent claims. It shall be understood that the claimed coil apparatus, apparatus and method have similar and/or identical preferred embodiments as the claimed cabling arrangement and as defined in the dependent claims.

Said "DC voltage" means a constant voltage with respect to said AC voltage, .i.e. this constant voltage may fluctuate or change but not significantly with respect to the AC voltage, over time.

Within MPI, the non-linearity of materials does not (or at least less) become effective if the material is driven into (or nearly into) magnetic saturation. The present invention applies this observation to the cabling, in particular of one or more coils, and proposes to apply dedicated DC currents to bring the magnetic contaminations of the cabling into saturation. In order not to generate an additional static magnetic field (which would shift the first sub-zone, i.e. the field-free point or field-free line), the DC currents are applied such that effectively no AC field, i.e. no far-field, is generated in the zone of interest. The static magnetic field is therefore mainly confined to within the cabling arrangement.

The magnitude of the DC current shall preferably be in the order of magnitude of the AC current (e.g. the drive field current) in order to substantially reduce the harmonics generated by the magnetic non-linearities within the cabling. Preferably, a considerable DC current is applied that leads to additional power dissipation in the conductor.

Due to the arrangement of the conductors of the first and second subsets the direction of the DC current is such that the conductors of the first subset receive the DC current in a first direction with respect to the area of interest and the conductors of the second subset receive the DC current in a second direction with respect to the area of interest, wherein the first and second directions are preferably opposite to each other.

Further, the present invention preferably takes an additional advantage of the DC current in tailoring the generated magnetic field such that the AC fields do not (nearly) cancel out in the entire field-of-view, but only cancel out in the center of the field-of-view, which is the first sub-zone. Hence, the field-free point or line is kept where it was, but additionally a magnetic selection field appears around the field-free point. This selection field can boost (or in some cases also replace) the magnetic selection field as generated by the dedicated selection field signal generator(s). This feature can thus be used to further increase the gradient of the selection field (which translates into better resolution), or to reduce power requirements on the dedicated selection field signal generator(s).

In summary, the present invention reduces the magnetic non-linearity of the cabling, used particularly in the drive field signal generator that leads to disturbing harmonic background in the detected signal. In the cabling arrangement, e.g. of the drive field means, different electrically parallel coils/lines that carry said AC currents, in which driven DC currents are additionally supplied (particularly from a DC generator) so as to bring the magnetic non-linearities into saturation. The DC currents applied to a first group of one or more conductors (e.g. coils) have opposite orientation to a second group of conductors (e.g. coils) in order not to generate AC magnetic fields.

According to a preferred embodiment the cabling arrangement comprises coupling elements coupled between the first AC terminal and the first internal terminal and between the first AC terminal and the second internal terminal,
wherein said coupling elements comprise at least a first capacitor coupled between the first AC terminal and the first internal terminal, and at least a second capacitor coupled between the first AC terminal and the second internal terminal. Said capacitors provide for splitting the first and second internal terminals so that they are at identical AC potential but at different DC potential.

According to another embodiment the first AC terminal is coupled to the first and second internal terminal. Hence, the DC voltage is applied in this embodiment either between the first AC terminal and the first internal terminal or between the first AC terminal and the second internal terminal.

Preferably, the cabling arrangement further a first capacitive arrangement coupled between the first AC terminal and the first and second internal terminals, and a second capacitive arrangement coupled between the second AC terminal and the first and second sets of conductors. Preferably, a first set of capacitors coupled to the first AC terminal, in particular in series, and/or a second set of capacitors coupled to the second AC terminal, in particular in series. These capacitors are provided to block the DC current from flowing through the first and/or second AC terminals.

Optionally, the cabling arrangement further comprises a first inductor coupled to the first internal terminal such that the first inductor and the first subset of conductors are located on either side of the first internal terminal and/or a second inductor coupled to the second internal terminal such that the second inductor and the second subset of conductors are located on either side of the second internal terminal; wherein the cabling arrangement is further configured for coupling said DC voltage to the first inductor and to the second inductor. The DC voltage source is then coupled between the first inductor and the second inductor, possibly via one or several switches. The purpose of this first and second inductor is to separate possible disturbances from the DC voltage source to enter the cabling arrangement, as well as to inhibit the AC voltage from entering the DC voltage source.

For the arrangement of the first and second conductors of the first and second subsets different options exist. In one embodiment the first subset comprises two or more first conductors and the second subset comprises two or more second conductors, wherein the first and second conductors are mechanically arranged (i.e. located) substantially in parallel thus forming a cable, wherein said cable is wound as a coil. Further, in one embodiment all first conductors are mechanically arranged (i.e. located) adjacent to each other and all second conductors are mechanically arranged (i.e. located) adjacent to each other, which provides a high saturation. In another embodiment the first conductors and the second conductors are mechanically arranged (i.e. located) alternately, which generally provides an even higher saturation and thus requires a reduced DC current and lower power.

Preferably, the cabling arrangement further comprises at least a third internal terminal and at least three coupling terminals coupled between the subsets and the second AC terminal, wherein a first end of the first conductors of the first subset are coupled to different internal terminals and a second end of the first conductors of the first subset are coupled to different coupling terminals, and wherein a first end of the second conductors of the second subset are coupled to different internal terminals and a second end of the second conductors of the second subset are coupled to different coupling terminals. This embodiment provides a higher resistance for the DC voltage. In other words, one or more pairs of conductors are provided, wherein each pair comprises one conductor from the first subset and one conductor from the second subset. Thereby, the two conductors of a pair experience a DC current of opposite direction with respect to the area of interest.

Preferably, in this embodiment the cabling arrangement further comprises a series coupling of one or more capacitors coupled between each internal terminal and the first AC terminal and/or a series coupling of one or more capacitors coupled between each coupling terminal and the second AC terminal. These capacitors are provided to block the DC current from flowing through the first and/or second AC terminals.

In another embodiment the first subset comprises a single first conductor wound as a first coil and the second subset comprises a single second conductor wound as a second coil, wherein the first coil and the second coil are arranged coaxially. Preferably this can be realised with both coils as solenoids, or with both coils as saddle coils. The coils are thus arranged and provided with DC currents such that the stationary magnetic fields are antiparallel and thus boost the existing magnetic selection field.

As mentioned above the coil apparatus may be applied in different kinds of apparatuses, in particular MPI apparatuses. The MPI apparatuses may be differently configured. In one embodiment the apparatus further comprises drive and receiving means comprising said drive field signal generator unit, a signal receiving unit and a drive-receiving coil, said drive-receiving coil being configured both for changing the position in space of the two sub-zones in the field of view by means of a magnetic drive field so that the magnetization of the magnetic material changes locally and for acquiring detection signals, which detection signals depend on the magnetization in the field of view, which magnetization is influenced by the change in the position in space of the first and second sub-zone, wherein said one or more coil apparatuses form said drive-receiving coil. In other embodiments the proposed idea can be used in an inductive coupling network (or parts of it) used for coupling a generator unit to the respective coils, e.g. for connecting the drive field generator unit with the drive field coils.

In still another embodiment the apparatus comprises separate coils for the receiving coil and the drive field coils and/or the apparatus comprises selection-and-focus means including said selection means for generating a magnetic selection-and-focus field having a pattern in space of its magnetic field strength such that the first sub-zone and the second sub-zone are formed in the field of view and for changing the position in space of the field of view within an examination area, said selection-and-focus means comprising at least one set of selection-and-focus field coils and a selection-and-focus field generator unit for generating selection-and-focus field currents to be provided to said at least one set of selection-and-focus field coils for controlling the generation of said magnetic selection-and-focus field.

### BRIEF DESCRIPTION OF THE DRAWINGS

These and other aspects of the invention will be apparent from and elucidated with reference to the embodiment(s) described hereinafter. In the following drawings
Fig. 1 shows a first embodiment of an MPI apparatus,
Fig. 2 shows an example of the selection field pattern produced by an apparatus as shown in Fig. 1,
Fig. 3 shows a second embodiment of an MPI apparatus,
Fig. 4 shows a third and a fourth embodiment of an MPI apparatus,
Fig. 5 shows a block diagram of an MPI apparatus according to the present invention,
Fig. 6 shows a circuit diagram of a first embodiment of a coil apparatus according to the present invention,
Fig. 7 shows a circuit diagram of a second embodiment of a coil apparatus according to the present invention,
Fig. 8 shows a circuit diagram of a third embodiment of a coil apparatus according to the present invention,
Fig. 9 shows a circuit diagram of an embodiment of sets of capacitors for used in a coil apparatus according to the present invention,
Fig. 10 shows various implementations of the arrangement of the conductors in a cable according to the present invention,
Fig. 11 shows a circuit diagram of a fourth embodiment of a coil apparatus according to the present invention,
Fig. 12 shows an arrangement of saddle coils using a cabling arrangement according to the present invention, and
Fig. 13 shows an arrangement of split solenoid coils using a cabling arrangement according to the present invention.

### DETAILED DESCRIPTION OF THE INVENTION

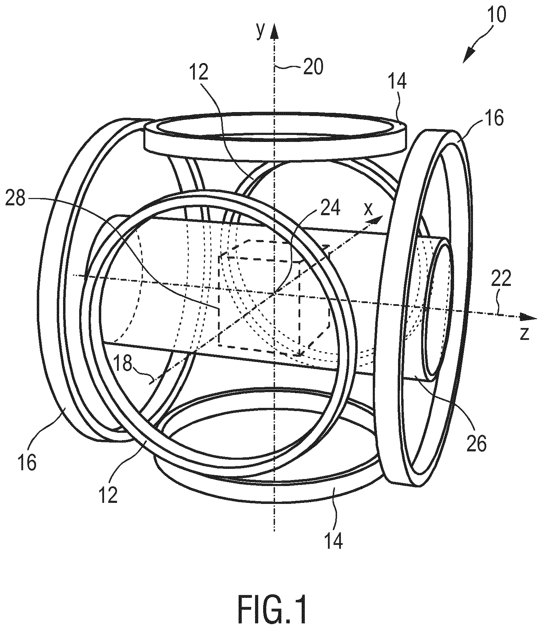

Before the details of the present invention shall be explained, basics of magnetic particle imaging shall be explained in detail with reference to Figs. 1 to 4. In particular, four embodiments of an MPI scanner for medical diagnostics will be described. An informal description of the data acquisition will also be given. The similarities and differences between the different embodiments will be pointed out. Generally, the present invention can be used in all these different embodiments of an MPI apparatus.

The first embodiment 10 of an MPI scanner shown in Fig. 1 has three pairs 12, 14, 16 of coaxial parallel circular coils, these coil pairs being arranged as illustrated in Fig. 1. These coil pairs 12, 14, 16 serve to generate the selection field as well as the drive and focus fields. The axes 18, 20, 22 of the three coil pairs 12, 14, 16 are mutually orthogonal and meet in a single point, designated the isocenter 24 of the MPI scanner 10. In addition, these axes 18, 20, 22 serve as the axes of a 3D Cartesian x-y-z coordinate system attached to the isocenter 24. The vertical axis 20 is nominated the y-axis, so that the x- and z-axes are horizontal. The coil pairs 12, 14, 16 are named after their axes. For example, the y-coilpair 14 is formed by the coils at the top and the bottom of the scanner. Moreover, the coil with the positive (negative) y-coordinate is called the y⁺-coil (y⁻-coil), and similarly for the remaining coils. When more convenient, the coordinate axes and the coils shall be labelled with x₁, x₂, and x₃, rather than with x, y, and z.

The scanner 10 can be set to direct a predetermined, time-dependent electric current through each of these coils 12, 14, 16, and in either direction. If the current flows clockwise around a coil when seen along this coil's axis, it will be taken as positive, otherwise as negative. To generate the static selection field, a constant positive current I^{S} is made to flow through the z⁺-coil, and the current -I^{S} is made to flow through the z⁻-coil. The z-coil pair 16 then acts as an anti-parallel circular coil pair.

It should be noted here that the arrangement of the axes and the nomenclature given to the axes in this embodiment is just an example and might also be different in other embodiments. For instance, in practical embodiments the vertical axis is often considered as the z-axis rather than the y-axis as in the present embodiment. This, however, does not generally change the function and operation of the device and the effect of the present invention.

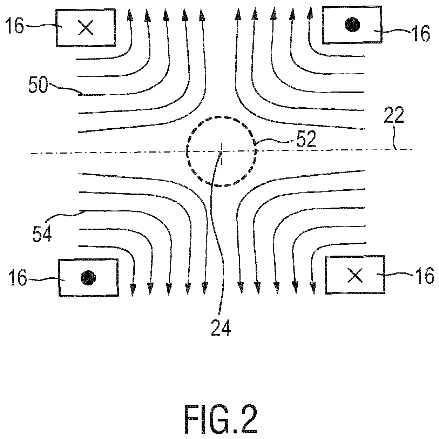

The magnetic selection field, which is generally a magnetic gradient field, is represented in Fig. 2 by the field lines 50. It has a substantially constant gradient in the direction of the (e.g. horizontal) z-axis 22 of the z-coil pair 16 generating the selection field and reaches the value zero in the isocenter 24 on this axis 22. Starting from this field-free point (not individually shown in Fig. 2), the field strength of the magnetic selection field 50 increases in all three spatial directions as the distance increases from the field-free point. In a first sub-zone or region 52 which is denoted by a dashed line around the isocenter 24 the field strength is so small that the magnetization of particles present in that first sub-zone 52 is not saturated, whereas the magnetization of particles present in a second sub-zone 54 (outside the region 52) is in a state of saturation. In the second sub-zone 54 (i.e. in the residual part of the scanner's field of view 28 outside of the first sub-zone 52) the magnetic field strength of the selection field is sufficiently strong to keep the magnetic particles in a state of saturation.

By changing the position of the two sub-zones 52, 54 (including the field-free point) within the field of view 28 the (overall) magnetization in the field of view 28 changes. By determining the magnetization in the field of view 28 or physical parameters influenced by the magnetization, information about the spatial distribution of the magnetic particles in the field of view 28 can be obtained. In order to change the relative spatial position of the two sub-zones 52, 54 (including the field-free point) in the field of view 28, further magnetic fields, i.e. the magnetic drive field, and, if applicable, the magnetic focus field, are superposed to the selection field 50.

To generate the drive field, a time dependent current I^{D}₁ is made to flow through both x-coils 12, a time dependent current I^{D}₂ through both y-coils 14, and a time dependent current I^{D}₃ through both z-coils 16. Thus, each of the three coil pairs acts as a parallel circular coil pair. Similarly, to generate the focus field, a time dependent current I^{F}₁ is made to flow through both x-coils 12, a current I^{F}₂ through both y-coils 14, and a current I^{F}₃ through both z-coils 16.

It should be noted that the z-coil pair 16 is special: It generates not only its share of the drive and focus fields, but also the selection field (of course, in other embodiments, separate coils may be provided). The current flowing through the z^{±}-coil is I^{D}₃ + I^{F}₃ ± I^{S}. The current flowing through the remaining two coil pairs 12, 14 is I^{D}ₖ + I^{F}ₖ, k = 1, 2. Because of their geometry and symmetry, the three coil pairs 12, 14, 16 are well decoupled. This is wanted.

Being generated by an anti-parallel circular coil pair, the selection field is rotationally symmetric about the z-axis, and its z-component is nearly linear in z and independent of x and y in a sizeable volume around the isocenter 24. In particular, the selection field has a single field-free point (FFP) at the isocenter. In contrast, the contributions to the drive and focus fields, which are generated by parallel circular coil pairs, are spatially nearly homogeneous in a sizeable volume around the isocenter 24 and parallel to the axis of the respective coil pair. The drive and focus fields jointly generated by all three parallel circular coil pairs are spatially nearly homogeneous and can be given any direction and strength, up to some maximum strength. The drive and focus fields are also time-dependent. The difference between the focus field and the drive field is that the focus field varies slowly in time and may have a large amplitude, while the drive field varies rapidly and has a small amplitude. There are physical and biomedical reasons to treat these fields differently. A rapidly varying field with a large amplitude would be difficult to generate and potentially hazardous to a patient.

In a practical embodiment the FFP can be considered as a mathematical point, at which the magnetic field is assumed to be zero. The magnetic field strength increases with increasing distance from the FFP, wherein the increase rate might be different for different directions (depending e.g. on the particular layout of the device). As long as the magnetic field strength is below the field strength required for bringing magnetic particles into the state of saturation, the particle actively contributes to the signal generation of the signal measured by the device; otherwise, the particles are saturated and do not generate any signal.

The embodiment 10 of the MPI scanner has at least one further pair, preferably three further pairs, of parallel circular coils, again oriented along the x-, y-, and z-axes. These coil pairs, which are not shown in Fig. 1, serve as receive coils. As with the coil pairs 12, 14, 16 for the drive and focus fields, the magnetic field generated by a constant current flowing through one of these receive coil pairs is spatially nearly homogeneous within the field of view and parallel to the axis of the respective coil pair. The receive coils are supposed to be well decoupled. The time-dependent voltage induced in a receive coil is amplified and sampled by a receiver attached to this coil. More precisely, to cope with the enormous dynamic range of this signal, the receiver samples the difference between the received signal and a reference signal. The transfer function of the receiver is non-zero from zero Hertz ("DC") up to the frequency where the expected signal level drops below the noise level. Alternatively, the MPI scanner has no dedicated receive coils. Instead the drive field transmit coils may be used as receive coils as is the case according one embodiment according to the present invention using combined drive-receiving coils.

The embodiment 10 of the MPI scanner shown in Fig. 1 has a cylindrical bore 26 along the z-axis 22, i.e. along the axis of the selection field. All coils are placed outside this bore 26. For the data acquisition, the patient (or object) to be imaged is placed in the bore 26 such that the patient's volume of interest - that volume of the patient (or object) that shall be imaged - is enclosed by the scanner's field of view 28 - that volume of the scanner whose contents the scanner can image. The patient (or object) is, for instance, placed on a patient table. The field of view 28 is a geometrically simple, isocentric volume in the interior of the bore 26, such as a cube, a ball, a cylinder or an arbitrary shape. A cubical field of view 28 is illustrated in Fig. 1.

The size of the first sub-zone 52 is dependent on the strength of the gradient of the magnetic selection field and on the field strength of the magnetic field required for saturation, which in turn depends on the magnetic particles. For a sufficient saturation of typical magnetic particles at a magnetic field strength of 80 A/m and a gradient (in a given space direction) of the field strength of the magnetic selection field amounting to 50x10³ A/m², the first sub-zone 52 in which the magnetization of the particles is not saturated has dimensions of about 1 mm (in the given space direction).

The patient's volume of interest is supposed to contain magnetic nanoparticles. Prior to the diagnostic imaging of, for example, a tumor, the magnetic particles are brought to the volume of interest, e.g. by means of a liquid comprising the magnetic particles which is injected into the body of the patient (object) or otherwise administered, e.g. orally, to the patient.

Generally, various ways for bringing the magnetic particles into the field of view exist. In particular, in case of a patient into whose body the magnetic particles are to be introduced, the magnetic particles can be administered by use of surgical and non-surgical methods, and there are both methods which require an expert (like a medical practitioner) and methods which do not require an expert, e.g. can be carried out by laypersons or persons of ordinary skill or the patient himself / herself. Among the surgical methods there are potentially non-risky and/or safe routine interventions, e.g. involving an invasive step like an injection of a tracer into a blood vessel (if such an injection is at all to be considered as a surgical method), i.e. interventions which do not require considerable professional medical expertise to be carried out and which do not involve serious health risks. Further, non-surgical methods like swallowing or inhalation can be applied.

Generally, the magnetic particles are pre-delivered or pre-administered before the actual steps of data acquisition are carried out. In embodiments, it is, however, also possible that further magnetic particles are delivered / administered into the field of view.

An embodiment of magnetic particles comprises, for example, a spherical substrate, for example, of glass which is provided with a soft-magnetic layer which has a thickness of, for example, 5 nm and consists, for example, of an iron-nickel alloy (for example, Permalloy). This layer may be covered, for example, by means of a coating layer which protects the particle against chemically and/or physically aggressive environments, e.g. acids. The magnetic field strength of the magnetic selection field 50 required for the saturation of the magnetization of such particles is dependent on various parameters, e.g. the diameter of the particles, the used magnetic material for the magnetic layer and other parameters.

In the case of e.g. a diameter of 10 µm with such magnetic particles, a magnetic field of approximately 800 A/m (corresponding approximately to a flux density of 1 mT) is then required, whereas in the case of a diameter of 100 µm a magnetic field of 80 A/m suffices. Even smaller values are obtained when a coating of a material having a lower saturation magnetization is chosen or when the thickness of the layer is reduced.

In practice, magnetic particles commercially available under the trade name Resovist (or similar magnetic particles) are often used, which have a core of magnetic material or are formed as a massive sphere and which have a diameter in the range of nanometers, e.g. 40 or 60 nm.

For further details of the generally usable magnetic particles and particle compositions, the corresponding parts of EP 1224542, WO 2004/091386, WO 2004/091390, WO 2004/091394, WO 2004/091395, WO 2004/091396, WO 2004/091397, WO 2004/091398, WO 2004/091408 are herewith referred to, which are herein incorporated by reference. In these documents more details of the MPI method in general can be found as well.

During the data acquisition, the x-, y-, and z-coil pairs 12, 14, 16 generate a position- and time-dependent magnetic field, the applied field. This is achieved by directing suitable currents through the field generating coils. In effect, the drive and focus fields push the selection field around such that the FFP moves along a preselected FFP trajectory that traces out the volume of scanning - a superset of the field of view. The applied field orientates the magnetic nanoparticles in the patient. As the applied field changes, the resulting magnetization changes too, though it responds nonlinearly to the applied field. The sum of the changing applied field and the changing magnetization induces a time-dependent voltage Vₖ across the terminals of the receive coil pair along the xₖ-axis. The associated receiver converts this voltage to a signal Sₖ, which it processes further.

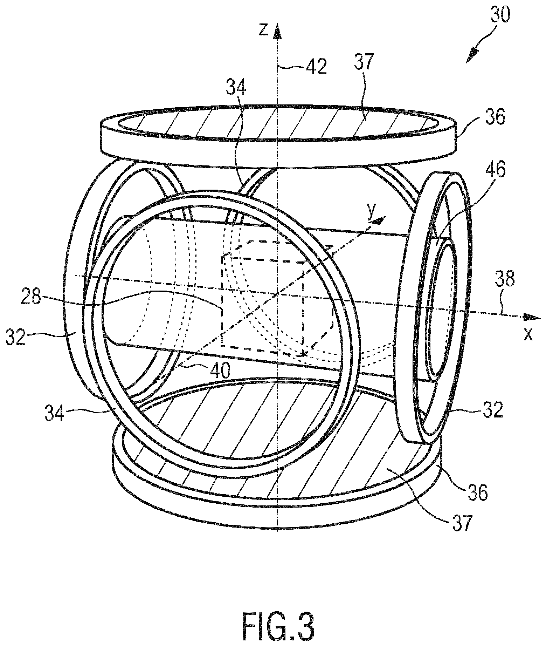

Like the first embodiment 10 shown in Fig. 1, the second embodiment 30 of the MPI scanner shown in Fig. 3 has three circular and mutually orthogonal coil pairs 32, 34, 36, but these coil pairs 32, 34, 36 generate the selection field and the focus field only. The z-coils 36, which again generate the selection field, are filled with ferromagnetic material 37. The z-axis 42 of this embodiment 30 is oriented vertically, while the x- and y-axes 38, 40 are oriented horizontally. The bore 46 of the scanner is parallel to the x-axis 38 and, thus, perpendicular to the axis 42 of the selection field. The drive field is generated by a solenoid (not shown) along the x-axis 38 and by pairs of saddle coils (not shown) along the two remaining axes 40, 42. These coils are wound around a tube which forms the bore. The drive field coils also serve as receive coils.

To give a few typical parameters of such an embodiment: The z-gradient of the selection field, G, has a strength of G/µ₀ = 2.5 T/m, where µ₀ is the vacuum permeability. The temporal frequency spectrum of the drive field is concentrated in a narrow band around 25 kHz (up to approximately 250 kHz). The useful frequency spectrum of the received signals lies between 50 kHz and 1 MHz (eventually up to approximately 15 MHz). The bore has a diameter of 120 mm. The biggest cube 28 that fits into the bore 46 has an edge length of 120 mm/√2 ≈ 84 mm.

Since the construction of field generating coils is generally known in the art, e.g. from the field of magnetic resonance imaging, this subject need not be further elaborated herein.

In an alternative embodiment for the generation of the selection field, permanent magnets (not shown) can be used. In the space between two poles of such (opposing) permanent magnets (not shown) there is formed a magnetic field which is similar to that shown in Fig. 2, that is, when the opposing poles have the same polarity. In another alternative embodiment, the selection field can be generated by a mixture of at least one permanent magnet and at least one coil.

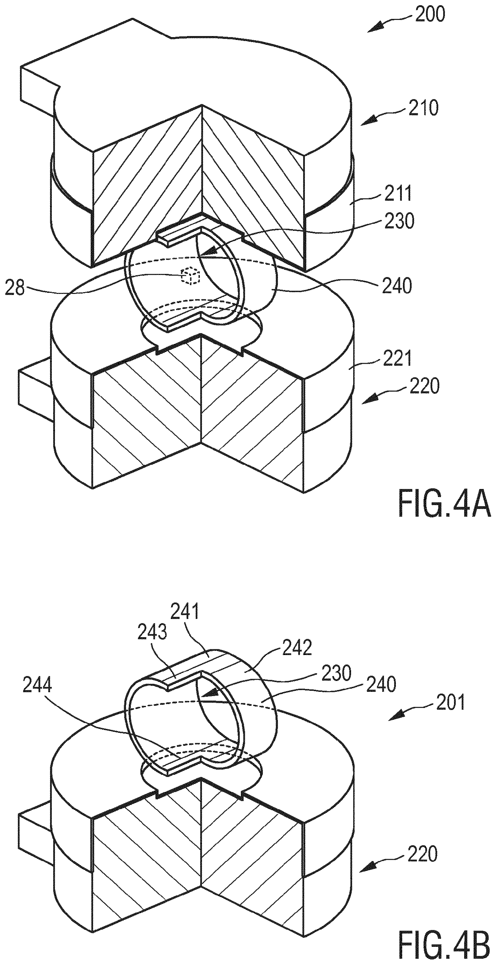

Fig. 4 shows two embodiments of the general outer layout of an MPI apparatus 200, 300. Fig. 4A shows an embodiment of the proposed MPI apparatus 200 comprising two selection-and-focus field coil units 210, 220 which are basically identical and arranged on opposite sides of the examination area 230 formed between them. Further, a drive field coil unit 240 is arranged between the selection-and-focus field coil units 210, 220, which are placed around the area of interest of the patient (not shown). The selection-and-focus field coil units 210, 220 comprise several selection-and-focus field coils for generating a combined magnetic field representing the above-explained magnetic selection field and magnetic focus field. In particular, each selection-and-focus field coil unit 210, 220 comprises a, preferably identical, set of selection-and-focus field coils. Details of said selection-and-focus field coils will be explained below.

The drive field coil unit 240 comprises a number of drive field coils for generating a magnetic drive field. These drive field coils may comprise several pairs of drive field coils, in particular one pair of drive field coils for generating a magnetic field in each of the three directions in space. In an embodiment the drive field coil unit 240 comprises two pairs of saddle coils for two different directions in space and one solenoid coil for generating a magnetic field in the longitudinal axis of the patient.

The selection-and-focus field coil units 210, 220 are generally mounted to a holding unit (not shown) or the wall of room. Preferably, in case the selection-and-focus field coil units 210, 220 comprise pole shoes for carrying the respective coils, the holding unit does not only mechanically hold the selection-and-focus field coil unit 210, 220 but also provides a path for the magnetic flux that connects the pole shoes of the two selection-and-focus field coil units 210, 220.

As shown in Fig. 4a, the two selection-and-focus field coil units 210, 220 each include a shielding layer 211, 221 for shielding the selection-and-focus field coils from magnetic fields generated by the drive field coils of the drive field coil unit 240.

In the embodiment of the MPI apparatus 201 shown in Fig. 4B only a single selection-and-focus field coil unit 220 is provided as well as the drive field coil unit 240. Generally, a single selection-and-focus field coil unit is sufficient for generating the required combined magnetic selection and focus field. Said single selection-and-focus field coil unit 220 may thus be integrated into a (not shown) patient table on which a patient is placed for the examination. Preferably, the drive field coils of the drive field coil unit 240 may be arranged around the patient's body already in advance, e.g. as flexible coil elements. In another implementation, the drive field coil unit 240 can be opened, e.g. separable into two subunits 241, 242 as indicated by the separation lines 243, 244 shown in Fig. 4b in axial direction, so that the patient can be placed in between and the drive field coil subunits 241, 242 can then be coupled together.

In still further embodiments of the MPI apparatus, even more selection-and-focus field coil units may be provided which are preferably arranged according to a uniform distribution around the examination area 230. However, the more selection-and-focus field coil units are used, the more will the accessibility of the examination area for placing a patient therein and for accessing the patient itself during an examination by medical assistance or doctors be limited.

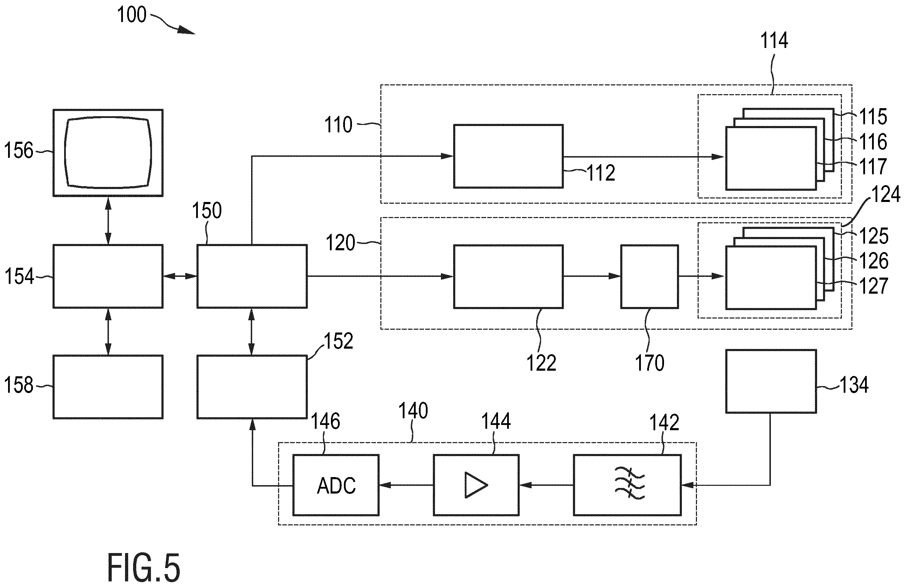

Fig. 5 shows a general block diagram of an MPI apparatus 100 according to the present invention. The general principles of magnetic particle imaging explained above are valid and applicable to this embodiment as well, unless otherwise specified.

The embodiment of the apparatus 100 shown in Fig. 5 comprises various coils for generating the desired magnetic fields. First, the coils and their functions in MPI shall be explained.

For generating the combined magnetic selection-and-focus field, selection-and-focus means 110 are provided. The magnetic selection-and-focus field has a pattern in space of its magnetic field strength such that the first sub-zone (52 in Fig. 2) having a low magnetic field strength where the magnetization of the magnetic particles is not saturated and a second sub-zone (54 in Fig. 4) having a higher magnetic field strength where the magnetization of the magnetic particles is saturated are formed in the field of view 28, which is a small part of the examination area 230, which is conventionally achieved by use of the magnetic selection field. Further, by use of the magnetic selection-and-focus field the position in space of the field of view 28 within the examination area 230 can be changed, as conventionally done by use of the magnetic focus field.

The selection-and-focus means 110 comprises at least one set of selection-and-focus field coils 114 and a selection-and-focus field generator unit 112 for generating selection-and-focus field currents to be provided to said at least one set of selection-and-focus field coils 114 (representing one of the selection-and-focus field coil units 210, 220 shown in Figs. 4A, 4B) for controlling the generation of said magnetic selection-and-focus field. Preferably, a separate generator subunit is provided for each coil element (or each pair of coil elements) of the at least one set of selection-and-focus field coils 114. Said selection-and-focus field generator unit 112 comprises a controllable current source (generally including an amplifier) and a filter unit which provide the respective coil element with the field current to individually set the gradient strength and field strength of the contribution of each coil to the magnetic selection-and-focus field. It shall be noted that the filter unit 114 can also be omitted. Further, separate focus and selection means are provided in other embodiments.

For generating the magnetic drive field the apparatus 100 further comprises drive means 120 comprising a drive field signal generator unit 122 and a set of drive field coils 124 (representing the drive coil unit 240 shown in Figs. 4A, 4B) for changing the position in space and/or size of the two sub-zones in the field of view by means of a magnetic drive field so that the magnetization of the magnetic material changes locally. As mentioned above said drive field coils 124 preferably comprise two pairs 125, 126 of oppositely arranged saddle coils and one solenoid coil 127. Other implementations, e.g. three pairs of coil elements, are also possible.

The drive field signal generator unit 122 preferably comprises a separate drive field signal generation subunit for each coil element (or at least each pair of coil elements) of said set of drive field coils 124. Said drive field signal generator unit 122 preferably comprises a drive field current source (preferably including a power amplifier) and a filter unit for providing a time-dependent drive field current to the respective drive field coil.

The selection-and-focus field signal generator unit 112 and the drive field signal generator unit 122 are preferably controlled by a control unit 150, which preferably controls the selection-and-focus field signal generator unit 112 such that the sum of the field strengths and the sum of the gradient strengths of all spatial points of the selection field is set at a predefined level. For this purpose the control unit 150 can also be provided with control instructions by a user according to the desired application of the MPI apparatus, which, however, is preferably omitted according to the present invention.

For using the MPI apparatus 100 for determining the spatial distribution of the magnetic particles in the examination area (or a region of interest in the examination area), particularly to obtain images of said region of interest, signal detection receiving means, in particular a receiving coil, and a signal receiving unit 140, which receives signals detected by said receiving means, are provided. One to three separate receiving coils 124 are provided in an MPI apparatus as receiving means.

According to other embodiments of the present invention, however, one to three of said drive field coils 124 (or drive field coil pairs) act (simultaneously or alternately) as receiving coils for receiving detection signals, wherein these drive field coils are then called "drive-receiving coils" herein. The generation of magnetic drive fields and the detection of detection signals may then be performed simultaneously or alternately. Preferably, all three drive-receiving coils (or coil pairs) may then act as receiving coils.

One to three receiving units 140 - one per receiving coil (or coil pair) - are provided in practice, but more than three receiving coils and receiving units can be also used, in which case the acquired detection signals are not 3-dimensional but K-dimensional, with K being the number of receiving coils.

Said signal receiving unit 140 comprises a filter unit 142 (also called Rx filter) for filtering the received detection signals. The aim of this filtering is to separate measured values, which are caused by the magnetization in the examination area which is influenced by the change in position of the two part-regions (52, 54), from other, interfering signals (in particular crosstalk of the fundamental frequency). To this end, the filter unit 142 may be designed for example such that signals which have temporal frequencies that are smaller than the temporal frequencies with which the drive coil(s) is (are) operated, or smaller than twice these temporal frequencies, do not pass the filter unit 142. The signals are then transmitted via an amplifier unit 144 (also called LNA, Low-Noise-Amplifier) to an analog/digital converter 146 (ADC).

The digitized signals produced by the analog/digital converter 146 are fed to an image processing unit (also called reconstruction means) 152, which reconstructs the spatial distribution of the magnetic particles from these signals and the respective position which the first part-region 52 of the first magnetic field in the examination area assumed during receipt of the respective signal and which the image processing unit 152 obtains from the control unit 150. The reconstructed spatial distribution of the magnetic particles is finally transmitted via the control means 150 to a computer 154, which displays it on a monitor 156. Thus, an image can be displayed showing the distribution of magnetic particles in the field of view of the examination area.

In other applications of the MPI apparatus 100, e.g. for influencing the magnetic particles (for instance for a hyperthermia treatment) or for moving the magnetic particles (e.g. attached to a catheter for moving the catheter or attached to a medicament for moving the medicament to a certain location) the receiving means may also be omitted or simply not used.

Further, an input unit 158 may optionally be provided, for example a keyboard. A user may therefore be able to set the desired direction of the highest resolution and in turn receives the respective image of the region of action on the monitor 156. If the critical direction, in which the highest resolution is needed, deviates from the direction set first by the user, the user can still vary the direction manually in order to produce a further image with an improved imaging resolution. This resolution improvement process can also be operated automatically by the control unit 150 and the computer 154. The control unit 150 in this embodiment sets the gradient field in a first direction which is automatically estimated or set as start value by the user. The direction of the gradient field is then varied stepwise until the resolution of the thereby received images, which are compared by the computer 154, is maximal, respectively not improved anymore. The most critical direction can therefore be found respectively adapted automatically in order to receive the highest possible resolution.

In MPI, the non-linearity of materials does not (or at least less) become effective if the material is driven into (or nearly into) magnetic saturation. The present invention applies this observation to the cabling, used particularly for the drive field coils 124, and applies dedicated DC currents to bring the magnetic contaminations of the cabling into saturation. In order not to generate an additional static magnetic field, which would shift the field-free point, the DC currents are applied such that effectively no AC (far-) field is generated. The static magnetic field is therefore mainly confined to within the cabling. The DC current shall be in the order of magnitude of the AC current (i.e. drive field) in order to substantially reduce the harmonics generated by the magnetic nonlinearities within the cabling.

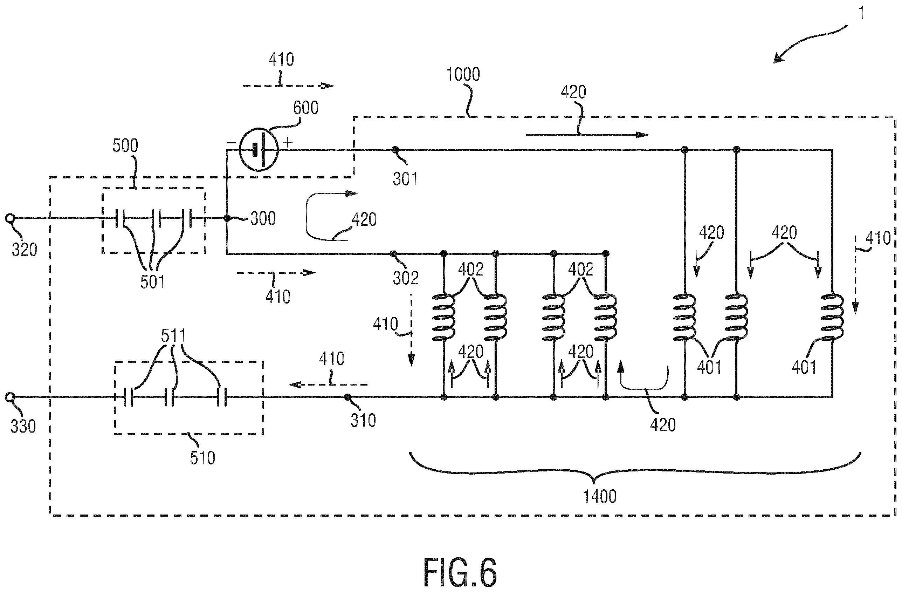

Fig. 6 shows a circuit diagram of a first embodiment of a coil apparatus 1 according to the present invention. The coil apparatus 1 comprises a first embodiment of a cabling arrangement 1000 and a DC voltage or current source 600 directly coupled between a first internal terminal 301 and a second internal terminal 302. The cabling arrangement 1000 comprises a first AC terminal 300, a second AC terminal 310, the first internal terminal 301 and the second internal terminal 302. A cabling 1400 (in particular conductors and/or inductors) is connected between the first internal terminal 301, the second internal terminal 302 and the second AC terminal 310. The cabling 1400 comprises a first subset of one or more first conductors 401 coupled between the first internal terminal 301 and the second AC terminal 310 and a second subset of one or more second conductors 402 coupled between the second internal terminal 302 and the second AC terminal 310. Further, the cabling arrangement 1000 is configured for coupling a DC voltage between the first internal terminal 301 and the second internal terminal 302, in this embodiment via the DC voltage (or current) source 600.

In one exemplary embodiment the cabling 1400 represents the conductors of the drive field coils (125, 126, and 127 in Fig. 5), which are preferably - due to the high current - realized by a plurality of parallel conductors, each e.g. consisting of 23000 x 20 µm Litz wires making up a Rutherford cable. These parallel conductors typically have considerable magnetic coupling. In such an embodiment the first and second conductors 401, 402 are mechanically arranged substantially in parallel and forming a cable, wherein said cable is wound as a coil.

Due to the high voltage, a first set 500 of capacitors 501 is coupled to the first AC terminal 300, in particular in series, and a second set 510 of capacitors 511 is coupled to the second AC terminal 310, in particular in series. To the other ends 320, 330 of the first and second sets 500, 510 of conductors the drive field signal generator unit (122 in Fig. 5) is coupled in case of using the present invention for implementing the drive field coils (124).

In the embodiment of the cabling 1400 presented in Fig. 6 the first subset of conductors 401 and the second subset of conductors 402 are mechanically essentially in parallel. The AC current (provided by the drive field generator) indicated by arrows 410 flows in parallel trough the conductors 401, 402. A DC current, indicated by arrows 420 additionally flows through them, but with a current direction within the conductors 401 being antiparallel to the current direction in the conductors 402. This way, the magnetic field essentially cancels out when observed from further away, wherein it is considered that the FOV is essentially in the far field.

The DC current flow is a result of a DC voltage applied by a voltage source 600, which is transparent to AC currents (or is made transparent by additional capacitors connected in parallel). This voltage source 600 can e.g. be a DC charged capacitor or electric battery. The positive side of the battery is connected to the first internal terminal 301 and the negative side of the battery is connected to the second internal terminal 302.

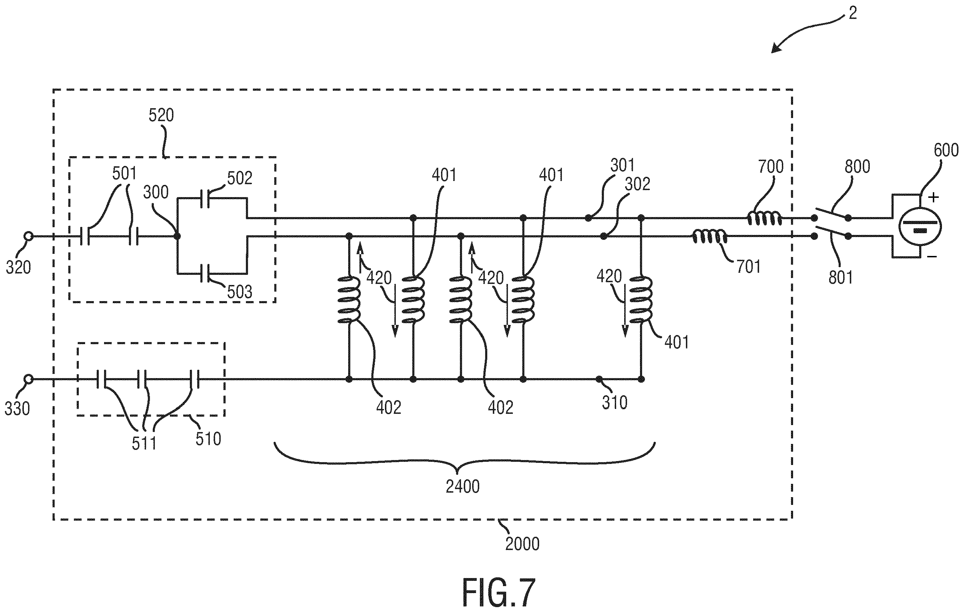

Fig. 7 shows a circuit diagram of a second embodiment of a coil apparatus 2 according to the present invention. The coil apparatus 2 comprises a second embodiment of a cabling arrangement 2000 including a cabling 2400. The DC current (or voltage) source 600 delivers its current to the internal terminals 301 and 302 via optional inductors 700, 701 and switches 800, 801. As in the first embodiment 1, the DC current source 600 might be a battery, an accumulator, a (rechargeable) (super)-capacitor, or a dedicated current source, or parallel connections of these, and is typically connected to further AC circuitry to receive its energy, optionally solely at intervals when no imaging takes places.

In order to separate internal terminal 301 from internal terminal 302, which are at different DC potential, but at identical AC potential, the last capacitor of the capacitors of the first set 520 is split into a first capacitor 502 and a second capacitor 503. Further, the conductors 401, 402 are alternately arranged, i.e. pairs of one first conductor 401 and one second conductor 402 are arranged adjacent to each other.

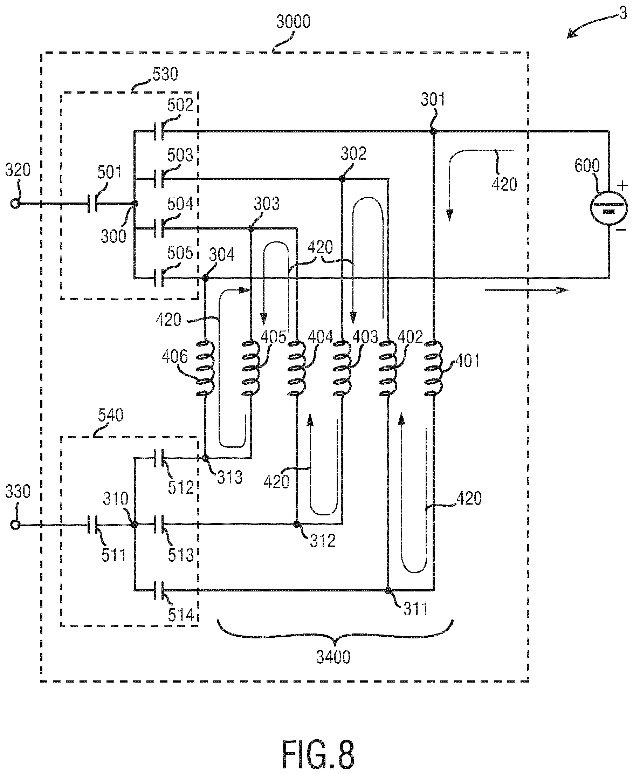

Fig. 8 shows a circuit diagram of a third embodiment of a coil apparatus 3 according to the present invention. The coil apparatus 3 comprises a third embodiment of a cabling arrangement 3000 including a cabling 3400. This embodiment provides a higher resistance to the voltage source 600, for an example with 6 parallel conductors 401 to 406.

In this embodiment a third internal terminal 303 and a fourth internal terminal 304 are provided. Further, three coupling terminals 311, 312, 313 are provided between the subsets of conductors 401-406 and the second AC terminal 310. A first end of the first conductors 401, 403, 405 of the first subset are coupled to different internal terminals 301, 302, 303 and a second end of the first conductors 401, 403, 405 of the first subset are coupled to different coupling terminals 311, 312, 313, and a first end of the second conductors 402, 404, 406 of the second subset are coupled to different internal terminals 302, 303, 304 and a second end of the second conductors 402, 404, 406 of the second subset are coupled to different coupling terminals 311, 312, 313.

The last capacitor of the capacitors of the first set 530 is split into four capacitors 502, 503, 504, 505, each coupled between the first AC terminal 300 and one of the internal terminals 301, 302, 303, 304. The second set 540 comprises a first capacitor 511 between the AC terminal 330 and the second AC terminal 310 and three capacitors 512, 513, 514, each coupled between the second AC terminal 310 and one of the coupling terminals 311,312,313.

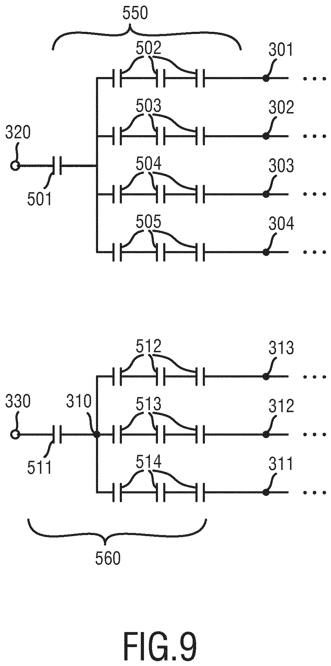

Fig. 9 shows a circuit diagram of an alternative embodiment of the first and second sets of capacitors, in particular for use in the third embodiment of the coil apparatus 3 shown in Fig. 8. The first set 550 is similar to the first set 530 shown in Fig. 8, wherein each of the capacitors 502, 503, 504, 505 is split into three (alternatively into two or four or more) capacitors. The second set 560 is similar to the second set 540 shown in Fig. 8, wherein each of the capacitors 512, 513, 514 is split into three (alternatively into two or four or more) capacitors. The embodiment provides a more equalized current flow around the peripheries of the capacitor and the cabling elements and hence reduces eddy current losses.

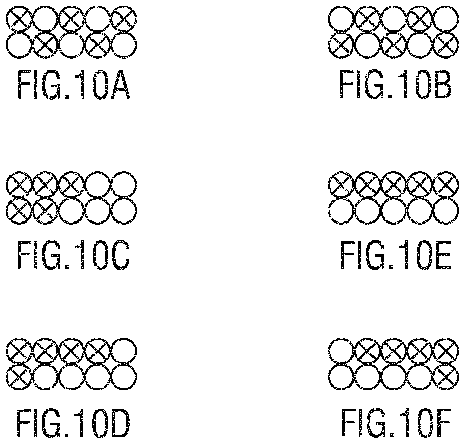

Fig. 10 shows various implementations of the cabling, i.e. the arrangement of the conductors in a cable according to the present invention. In particular, the cross sections of the cabling, which consists of parallel conductors that carry AC currents in alike, but DC currents of opposite direction, are shown. The conductor with DC currents flowing into the paper plane are marked by "x" and are termed 402. The remaining unmarked conductors with currents flowing out of the paper plane are termed 403. The layout of the conductors shown in the cross section shall be exemplarily for the way the cabling is shaped inside the drive field coil. Various assignments of current direction to the individual positions are possible.

Figs. 10A and 10B show an arrangement in which alternatingly each second conductor has the same current direction. Due to (possible, but not necessary) rotation of the cable (which could e.g. be formed as a Rutherford cable), the position changes along the length of the cabling. So the cross section of the cabling as shown in Fig. 10A will become as shown in Fig. 10B at a different position in the cabling.

Figs. 10C to 10F show arrangements in which the conductors of identical current direction are clustered. The benefit of the clustering might be to have, on average, a stronger saturation, which might be translated to reduced DC currents and hence power requirements.

The present invention reduces the magnetic non-linearity of the cabling, used particularly in the drive field coils, that leads to disturbing harmonic background in the detected signal. In preferred embodiments additional advantage is taken of the DC current in tailoring the generated magnetic field such that the AC fields do not (nearly) cancel out in the entire field-of-view but only cancel out in the center of the field-of-view, which is the field-free point. Hence, the field-free point is kept where it was, but additionally a "selection field" appears around the field-free point. This selection field can boost (or theoretically (in some cases) also replace) the selection field as generated by dedicated selection field coils. This feature can be used to further increase the gradient of the selection field (which translates into better resolution), or to reduce power requirements on the dedicated selection field coils.

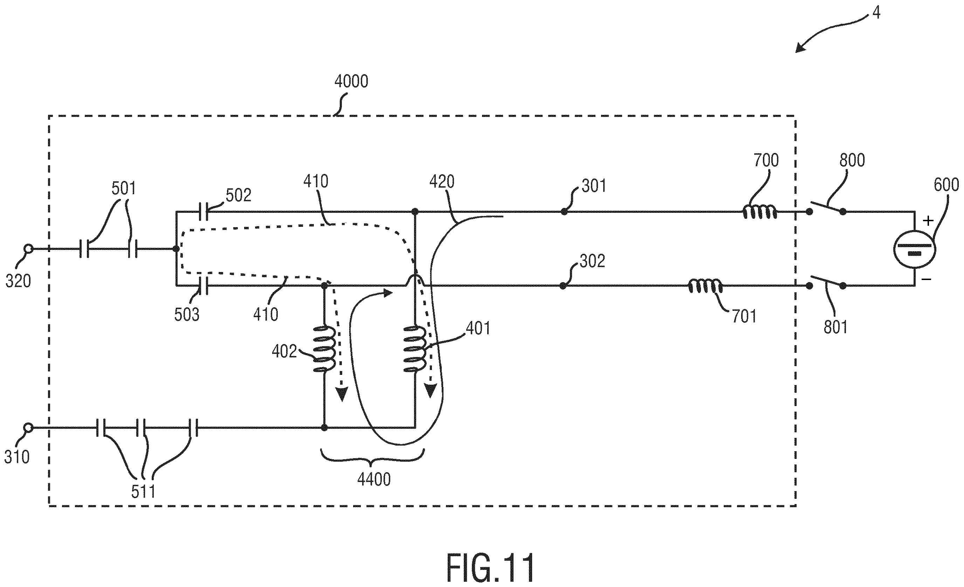

Fig. 11 shows a circuit diagram of a fourth embodiment of a coil apparatus 4 according to the present invention. The coil apparatus 4 comprises a fourth embodiment of a cabling arrangement 4000 including a cabling 4400. The coil apparatus 4 is rather similar to the second embodiment of the coil apparatus 2 shown in Fig. 7, but has just two different conductors 401, 402 in parallel. The AC and DC currents have anti-parallel directions in coil 402 and parallel directions in coil 401.

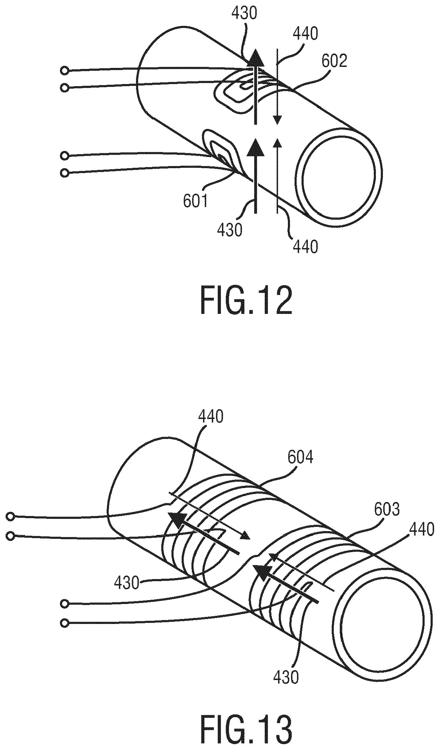

Fig. 12 shows an arrangement of saddle coils using a cabling arrangement according to the present invention, especially as the cabling 4400 of Fig. 11. In particular a saddle coil pair of two saddle coils 601, 602, which is used as drive field coils for both the z-(anterior-posterior) and the y-(left-right) direction), is formed, wherein coil 601 is formed by the conductor 401 and coil 602 is formed by the conductor 402. It is shown how the magnetic fields 430 from the AC current add up in the entire field-of-view. The magnetic fields 440 from the DC currents oppose, i.e. at the very center, where the field-free point resides, no static field is superposed. However, when approaching conductor 402 (top coil 602) or conductor 401 (bottom coil 601), the magnetic field increases, which is exactly the property of the selection field.

Fig. 13 shows an arrangement of split solenoid coils using a cabling arrangement according to the present invention, especially as the cabling 4400 of Fig. 11. In particular a solenoid coil of two solenoid coils 603 (formed by conductor 401), 604 (formed by conductor 402) is shown, which is traditionally realized as one coil and split into two halves here. This coil is used for generating AC fields in the x-direction (head-feet). Since the AC selection field has a fixed orientation, e.g. in the z- (anterior-posterior) direction, as drawn in Fig. 12, the selection field generated by the split solenoid coils is different (i.e. orthogonal) in orientation. However, the x-direction (head-feet) selection field from the split solenoid coils is complemented by the (also) different (orthogonal) selection field from the other saddle coil pairs in the y-(left-right)-direction, which in sum generates a selection field in the desired direction (z-, anterior-posterior).

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A cabling arrangement for generating a magnetic field, in particular for use in a magnetic particle imaging apparatus (100), comprising:
- a first AC terminal (320),
- a second AC terminal (330), wherein said first and second AC terminals are configured for coupling an AC voltage between them,
- a first internal terminal (301),
- a second internal terminal (302), wherein said first and second internal terminals are configured for coupling a DC voltage between them,
- a first subset of one or more first conductors (401) coupled between the first internal terminal and the second AC terminal, and
- a second subset of one or more second conductors (402) coupled between the second internal terminal and the second AC terminal,
wherein the cabling arrangement enables the superposition of the AC and DC voltages in said first and second conductors, and wherein said first and second conductors are arranged to form a coil (125, 126, 127; 601, 602, 603, 604) to generate a magnetic field in a zone of interest.

2. The cabling arrangement as claimed in claim 1, further comprising coupling elements (501, 502, 503) coupled between the first AC terminal and the first internal terminal and between the first AC terminal and the second internal terminal,
wherein said coupling elements comprise:
- at least a first capacitor (502) coupled between the first AC terminal (320) and the first internal terminal (301), and
- at least a second capacitor (503) coupled between the first AC terminal (320) and the second internal terminal (302).

3. The cabling arrangement as claimed in claim 1,
wherein the first AC terminal (300) is coupled to the first and the second internal terminal (301, 302).

4. The cabling arrangement as claimed in claim 1, further comprising
- a first capacitive arrangement (500) coupled between the first AC terminal and the first and second internal terminals, and
- a second capacitive arrangement (510) coupled between the second AC terminal and the first and second sets of conductors.

5. The cabling arrangement as claimed in claim 1, further comprising
- a first inductor (700) coupled to the first internal terminal such that the first inductor and the first subset of conductors are located on either side of the first internal terminal and/or
- a second inductor (701) coupled to the second internal terminal such that the second inductor and the second subset of conductors are located on either side of the second internal terminal,
wherein the cabling arrangement is further configured for coupling said DC voltage to the first inductor and to the second inductor.

6. The cabling arrangement as claimed in claim 1,
wherein the first subset comprises two or more first conductors (401) and the second subset comprises two or more second conductors (402),
wherein the first and second conductors are mechanically arranged substantially in parallel forming a cable, wherein said cable is wound as a coil.

7. The cabling arrangement as claimed in claim 6,
wherein all first conductors (401) are mechanically arranged adjacent to each other and all second conductors (402) are mechanically arranged adjacent to each other.

8. The cabling arrangement as claimed in claim 6,
wherein the first conductors (401) and the second conductors (402) are mechanically arranged alternately.

9. The cabling arrangement as claimed in claim 6, further comprising
- at least a third internal terminal (303) and
- at least three coupling terminals (311, 312, 313) coupled between the first and second subsets and the second AC terminal,
wherein first ends of the first conductors of the first subset are coupled to different internal terminals and second ends of the first conductors of the first subset are coupled to different coupling terminals, and
wherein first ends of the second conductors of the second subset are coupled to different internal terminals and second ends of the second conductors of the second subset are coupled to different coupling terminals.

10. The cabling arrangement as claimed in claim 9, further comprising
- a series coupling of one or more capacitors (502, 503, 504, 505) coupled between each internal terminal and the first AC terminal and/or
- a series coupling of one or more capacitors (512, 513, 514) coupled between each coupling terminal and the second AC terminal.

11. The cabling arrangement as claimed in claim 1,
wherein the first subset comprises a single first conductor (401) wound as a first coil (601, 603) and the second subset comprises a single second conductor (402) wound as a second coil (602, 604), wherein the first coil and the second coil are arranged coaxially.

12. A coil apparatus, in particular for use in a magnetic particle imaging apparatus (100), comprising:
- a cabling arrangement (1000, 2000, 3000, 4000) as claimed in claim 1 and
- a DC voltage or current source (600) coupled, in particular via switches (800, 801), between the first and second internal terminals (301, 302).

13. An apparatus (100) for influencing and/or detecting magnetic particles in a field of view (28), which apparatus comprises:
- selection means comprising a selection field signal generator unit (110) and selection field elements (116) for generating a magnetic selection field (50) having a pattern in space of its magnetic field strength such that a first sub-zone (52) having a low magnetic field strength where the magnetization of the magnetic particles is not saturated and a second sub-zone (54) having a higher magnetic field strength where the magnetization of the magnetic particles is saturated are formed in the field of view (28),
- drive means (120) comprising a drive field signal generator unit (122) and drive field coils (124; 125, 126, 127) for changing the position in space of the two sub-zones (52, 54) in the field of view (28) by means of a magnetic drive field so that the magnetization of the magnetic material changes locally, and
- one or more coil apparatuses (1, 2, 3, 4) as claimed in claim 12 forming said drive field coils, wherein the drive field signal generator unit is coupled to the AC terminals of the one or more cabling arrangements of the one or more coil apparatuses.

14. The apparatus as claimed in claim 13,
further comprising drive and receiving means comprising said drive field signal generator unit (122), a signal receiving unit (140) and a drive-receiving coil (124), said drive-receiving coil being configured both for changing the position in space of the two sub-zones (52, 54) in the field of view (28) by means of a magnetic drive field so that the magnetization of the magnetic material changes locally and for acquiring detection signals, which detection signals depend on the magnetization in the field of view (28), which magnetization is influenced by the change in the position in space of the first and second sub-zone (52, 54), wherein said one or more coil apparatuses form said drive-receiving coil.

15. A method for influencing and/or detecting magnetic particles in a field of view (28), which method comprises:
- generating a magnetic selection field (50) having a pattern in space of its magnetic field strength such that a first sub-zone (52) having a low magnetic field strength where the magnetization of the magnetic particles is not saturated and a second sub-zone (54) having a higher magnetic field strength where the magnetization of the magnetic particles is saturated are formed in the field of view (28),
- changing the position in space of the two sub-zones (52, 54) in the field of view (28) by means of a magnetic drive field so that the magnetization of the magnetic material changes locally, and
- forming said drive field coils by one or more coil apparatuses (1, 2, 3, 4) as claimed in claim 12,
- applying DC currents to drive field coils formed by one or more cabling arrangements (1000, 2000, 3000, 4000) as claimed in claim 1 and used to generate the magnetic drive field, said DC currents being configured to bring magnetic contaminations of the first and second conductors of the one or more cabling arrangements into saturation and to generate no additional static magnetic field or only in the first sub-zone (52).

## Patentansprüche

1. Verkabelungsanordnung zur Erzeugung eines Magnetfeldes, vorzugsweise zur Verwendung in einer Magnetpartikelbildgebungsvorrichtung (100), umfassend:
- einen ersten AC-Anschluss (320),
- einen zweiten AC-Anschluss (330), wobei der erste und zweite AC-Anschluss zur Kopplung einer AC-Spannung zwischen diesen ausgeführt sind,
- einen ersten Innenanschluss (301),
- einen zweiten Innenanschluss (302), wobei der erste und zweite Innenanschluss zur Kopplung einer DC-Spannung zwischen diesen ausgeführt sind,
- eine erste Teilmenge von einem oder mehreren ersten Leitern (401), die zwischen dem ersten Innenanschluss und dem zweiten AC-Anschluss gekoppelt sind, sowie
- eine zweite Teilmenge von einem oder mehreren zweiten Leitern (402), die zwischen dem zweiten Innenanschluss und dem zweiten AC-Anschluss gekoppelt sind, wobei die Verkabelungsanordnung die Überlagerung der AC- und DC-Spannung in dem ersten und zweiten Leiter ermöglicht, und wobei der erste und zweite Leiter so angeordnet sind, dass sie eine Spule (125, 126, 127; 601, 602, 603, 604) zur Erzeugung eines Magnetfeldes in einer interessierenden Zone bilden.

2. Verkabelungsanordnung nach Anspruch 1, weiterhin umfassend Koppelelemente (501, 502, 503), die zwischen dem ersten AC-Anschluss und dem ersten Innenanschluss und zwischen dem ersten AC-Anschluss und dem zweiten Innenanschluss gekoppelt sind,
wobei die Koppelelemente umfassen:
- zumindest einen ersten Kondensator (502), der zwischen dem ersten AC-Anschluss (320) und dem ersten Innenanschluss (301) gekoppelt ist, sowie
- zumindest einen zweiten Kondensator (503), der zwischen dem ersten AC-Anschluss (320) und dem zweiten Innenanschluss (302) gekoppelt ist.

3. Verkabelungsanordnung nach Anspruch 1,
wobei der erste AC-Anschluss (300) mit dem ersten und dem zweiten Innenanschluss (301, 302) gekoppelt ist.

4. Verkabelungsanordnung nach Anspruch 1, weiterhin umfassend:
- eine erste kapazitive Anordnung (500), die zwischen dem ersten AC-Anschluss und dem ersten und zweiten Innenanschluss gekoppelt ist, sowie
- eine zweite kapazitive Anordnung (510), die zwischen dem zweiten AC-Anschluss und dem ersten und zweiten Satz Leitern gekoppelt ist.

5. Verkabelungsanordnung nach Anspruch 1, weiterhin umfassend:
- einen ersten Induktor (700), der mit dem ersten Innenanschluss so gekoppelt ist, dass der erste Induktor und die erste Teilmenge von Leitern auf beiden Seiten des ersten Innenanschlusses angeordnet sind und/oder
- einen zweiten Induktor (701), der mit dem zweiten Innenanschluss so gekoppelt ist, dass der zweite Induktor und die zweite Teilmenge von Leitern auf beiden Seiten des zweiten Innenanschlusses angeordnet sind,
wobei die Verkabelungsanordnung weiterhin zur Kopplung der DC-Spannung mit dem ersten Induktor und mit dem zweiten Induktor konfiguriert ist.

6. Verkabelungsanordnung nach Anspruch 1,
wobei die erste Teilmenge zwei oder mehrere erste Leiter (401) und die zweite Teilmenge zwei oder mehrere zweite Leiter (402) umfasst,
wobei die ersten und zweiten Leiter zur Ausbildung eines Kabels im Wesentlichen parallel mechanisch angeordnet sind, wobei das Kabel als eine Spule gewickelt ist.

7. Verkabelungsanordnung nach Anspruch 6,
wobei alle ersten Leiter (401) in Angrenzung aneinander mechanisch angeordnet sind und alle zweiten Leiter (402) in Angrenzung aneinander mechanisch angeordnet sind.

8. Verkabelungsanordnung nach Anspruch 6,
wobei die ersten Leiter (401) und die zweiten Leiter (402) abwechselnd mechanisch angeordnet sind.

9. Verkabelungsanordnung nach Anspruch 6, weiterhin umfassend:
- zumindest einen dritten Innenanschluss (303) sowie
- mindestens drei Koppelanschlüsse (311, 312, 313), die zwischen der ersten und zweiten Teilmenge und dem zweiten AC-Anschluss gekoppelt sind,
wobei erste Enden der ersten Leiter der ersten Teilmenge mit verschiedenen Innenanschlüssen und zweite Enden der ersten Leiter der ersten Teilmenge mit verschiedenen Koppelanschlüssen gekoppelt sind, und
wobei erste Enden der zweiten Leiter der zweiten Teilmenge mit verschiedenen Innenanschlüssen und zweite Enden der zweiten Leiter der zweiten Teilmenge mit verschiedenen Koppelanschlüssen gekoppelt sind.

10. Verkabelungsanordnung nach Anspruch 9, weiterhin umfassend:
- eine Reihenschaltung von einem oder mehreren Kondensatoren (502, 503, 504, 505), die zwischen jedem Innenanschluss und dem ersten AC-Anschluss gekoppelt sind und/oder
- eine Reihenschaltung von einem oder mehreren Kondensatoren (512, 513, 514), die zwischen jedem Koppelanschluss und dem zweiten AC-Anschluss gekoppelt sind.

11. Verkabelungsanordnung nach Anspruch 1,
wobei die erste Teilmenge einen als eine erste Spule (601, 603) gewickelten, einzelnen ersten Leiter (401) umfasst und die zweite Teilmenge einen als eine zweite Spule (602, 604) gewickelten, einzelnen zweiten Leiter (402) umfasst, wobei die erste Spule und die zweite Spule koaxial angeordnet sind.

12. Spuleneinrichtung, vorzugsweise zur Verwendung in einer Magnetpartikelbildgebungsvorrichtung (100), umfassend:
- eine Verkabelungsanordnung (1000, 2000, 3000, 4000) nach Anspruch 1 sowie
- eine DC-Spannungs- oder Stromquelle (600), die vorzugsweise über Schalter (800, 801) zwischen dem ersten und zweiten Innenanschluss (301, 302) gekoppelt ist.

13. Vorrichtung (100), um Magnetpartikel in einem Sichtfeld (28) zu beeinflussen und/oder zu detektieren, wobei die Vorrichtung umfasst:
- Auswahlmittel mit einer Auswahlfeld-Signalgeneratoreinheit (110) und Auswahlfeldelementen (116), um ein magnetisches Auswahlfeld (50) mit einem räumlichen Muster seiner Magnetfeldstärke so zu erzeugen, dass eine erste Teilzone (52) mit einer niedrigen Magnetfeldstärke, in der die Magnetisierung der Magnetpartikel nicht gesättigt ist, und eine zweite Teilzone (54) mit einer höheren Magnetfeldstärke, in der die Magnetisierung der Magnetpartikel gesättigt ist, in dem Sichtfeld (28) gebildet werden,
- Ansteuerungsmittel (120) mit einer Ansteuerungsfeld-Signalgeneratoreinheit (122) und Ansteuerungsfeldspulen (124; 125, 126, 127), um die räumliche Position der zwei Teilzonen (52, 54) in dem Sichtfeld (28) mit Hilfe eines magnetischen Ansteuerungsfeldes so zu verändern, dass sich die Magnetisierung des magnetischen Materials lokal verändert, sowie
- eine oder mehrere Spuleneinrichtungen (1, 2, 3, 4) nach Anspruch 12, welche die Ansteuerungsfeldspulen bilden, wobei die Ansteuerungsfeld-Signalgeneratoreinheit mit den AC-Anschlüssen der einen oder mehrerer Verkabelungsanordnungen der einen oder mehrerer Spuleneinrichtungen gekoppelt ist.

14. Vorrichtung nach Anspruch 13,
weiterhin umfassend Ansteuerungs- und Empfangsmittel mit der Ansteuerungsfeld-Signalgeneratoreinheit (122), einer Signalempfangseinheit (140) und einer Ansteuerungs-Empfangsspule (124), wobei diese Ansteuerungs-Empfangsspule so ausgeführt ist, dass sie sowohl die räumliche Position der beiden Teilzonen (52, 54) in dem Sichtfeld (28) mit Hilfe eines magnetischen Ansteuerungsfeldes so verändert, dass sich die Magnetisierung des magnetischen Materials lokal verändert, als auch Detektionssignale erfasst, wobei die Detektionssignale von der Magnetisierung in dem Sichtfeld (28) abhängen, wobei die Magnetisierung durch die Änderung der räumlichen Position der ersten und zweiten Teilzone (52, 54) beeinflusst wird, wobei diese eine oder mehrere Spuleneinrichtungen die Ansteuerungs-Empfangsspule bilden.

15. Verfahren, um Magnetpartikel in einem Sichtfeld (28) zu beeinflussen und/oder zu detektieren, wobei gemäß dem Verfahren:
- ein magnetisches Auswahlfeld (50) mit einem räumlichen Muster seiner Magnetfeldstärke so erzeugt wird, dass eine erste Teilzone (52) mit einer niedrigen Magnetfeldstärke, in der die Magnetisierung der Magnetpartikel nicht gesättigt ist, und eine zweite Teilzone (54) mit einer höheren Magnetfeldstärke, in der die Magnetisierung der Magnetpartikel gesättigt ist, in dem Sichtfeld (28) gebildet werden,
- die räumliche Position der zwei Teilzonen (52, 54) in dem Sichtfeld (28) mit Hilfe eines magnetischen Ansteuerungsfeldes so verändert wird, dass sich die Magnetisierung des magnetischen Materials lokal verändert, und
- die Ansteuerungsfeldspulen von einer oder mehreren Spuleneinrichtungen (1, 2, 3, 4) nach Anspruch 12 gebildet werden,
- DC-Ströme an Ansteuerungsfeldspulen angelegt werden, die von einer oder mehreren Verkabelungsanordnungen (1000, 2000, 3000, 4000) nach Anspruch 1 gebildet und zur Erzeugung des magnetischen Ansteuerungsfeldes verwendet werden, wobei die DC-Ströme so vorgesehen sind, dass sie magnetische Kontaminationen der ersten und zweiten Leiter der einen oder mehrerer Verkabelungsanordnungen in Sättigung bringen und kein zusätzliches statisches Magnetfeld oder nur in der ersten Teilzone (52) erzeugen.

## Revendications

1. Agencement de câblage pour générer un champ magnétique, en particulier destiné à être utilisé dans un appareil d'imagerie à particules magnétiques (100) comprenant :
- une première borne CA (320),
- une seconde borne CA (330), dans lequel lesdites première et seconde bornes CA sont configurées pour coupler une tension CA entre elles,
- une première borne interne (301),
- une deuxième borne interne (302), dans lequel lesdites première et deuxième bornes internes sont configurées pour coupler une tension CC entre elles,
- un premier sous-ensemble d'un ou plusieurs premiers conducteurs (401) couplés entre la première borne interne et la seconde borne CA, et
- un second sous-ensemble d'un ou plusieurs seconds conducteurs (402) couplés entre la deuxième borne interne et la seconde borne CA,
dans lequel l'agencement de câblage permet la superposition de tensions CA et CC dans lesdits premiers et seconds conducteurs, et dans lequel lesdits premiers et seconds conducteurs sont agencés pour former une bobine (125, 126, 127 ; 601, 602, 603, 604) pour générer un champ magnétique dans une zone d'intérêt.

2. Agencement de câblage selon la revendication 1, comprenant en outre des éléments de couplage (501, 502, 503) couplés entre la première borne CA et la première borne interne et entre la première borne CA et la deuxième borne interne,
dans lequel lesdits éléments de couplage comprennent :
- au moins un premier condensateur (502) couplé entre la première borne CA (320) et la première borne interne (301), et
- au moins un second condensateur (503) couplé entre la première borne CA (320) et la deuxième borne interne (302).

3. Agencement de câblage selon la revendication 1,
dans lequel la première borne CA (300) est couplée aux première et deuxième bornes internes (301, 302).

4. Agencement de câblage selon la revendication 1, comprenant en outre
- un premier agencement capacitif (500) couplé entre la première borne CA et les première et deuxième bornes internes, et
- un second agencement capacitif (510) couplé entre la seconde borne CA et les premier et second ensembles de conducteurs.

5. Agencement de câblage selon la revendication 1, comprenant en outre
- un premier inducteur (700) couplé à la première borne interne de sorte que le premier inducteur et le premier sous-ensemble de conducteurs soient situés de chaque côté de la première borne interne et/ou
- un second inducteur (701) couplé à la deuxième borne interne de sorte que le second inducteur et le second sous-ensemble de conducteurs soient situés de chaque côté de la deuxième borne interne,
dans lequel l'agencement de câblage est en outre configuré pour coupler ladite tension CC au premier inducteur et au second inducteur.

6. Agencement de câblage selon la revendication 1,
dans lequel le premier sous-ensemble comprend au moins deux premiers conducteurs (401) et le second sous-ensemble comprend au moins deux seconds conducteurs (402),
dans lequel les premiers et seconds conducteurs sont agencés mécaniquement sensiblement en parallèle pour former un câble, dans lequel ledit câble est enroulé à la manière d'une bobine.

7. Agencement de câblage selon la revendication 6,
dans lequel tous les premiers conducteurs (401) sont agencés mécaniquement adjacents les uns par rapport aux autres et tous les seconds conducteurs (402) sont agencés mécaniquement adjacents les uns par rapport aux autres.

8. Agencement de câblage selon la revendication 6,
dans lequel les premiers conducteurs (401) et les seconds conducteurs (402) sont agencés mécaniquement en alternance.

9. Agencement de câblage selon la revendication 6, comprenant en outre
- au moins une troisième borne interne (303) et
- au moins trois bornes de couplage (311, 312, 313) couplées entre les premier et second sous-ensembles et la seconde borne CA,
dans lequel des premières extrémités des premiers conducteurs du premier sous-ensemble sont couplées à différentes bornes internes et des secondes extrémités des premiers conducteurs du premier sous-ensemble sont couplées à différentes bornes de couplage, et dans lequel des premières extrémités des seconds conducteurs du second sous-ensemble sont couplées à différentes bornes internes et des secondes extrémités des seconds conducteurs du second sous-ensemble sont couplées à différentes bornes de couplage.

10. Agencement de câblage selon la revendication 9, comprenant en outre
- une série de couplage d'un ou plusieurs condensateurs (502, 503, 504, 505) couplés entre chaque borne interne et la première borne CA et/ou
- une série de couplage d'un ou plusieurs condensateurs (512, 513, 514) couplés entre chaque borne de couplage et la seconde borne CA.

11. Agencement de câblage selon la revendication 1,
dans lequel le premier sous-ensemble comprend un seul premier conducteur (401) enroulé à la manière d'une première bobine (601, 603) et le second sous-ensemble comprend un seul second conducteur (402) enroulé à la manière d'une seconde bobine (602, 604), dans lequel la première bobine et la seconde bobine sont agencées coaxialement.

12. Appareil à bobines, en particulier destiné à être utilisé dans un appareil d'imagerie à particules magnétiques (100), comprenant :
- un agencement de câblage (1000, 2000, 3000, 4000) selon la revendication 1 et
- une source de tension ou de courant CC (600) couplée, en particulier par l'intermédiaire d'interrupteurs (800, 801) entre les première et deuxième bornes internes (301, 302).

13. Appareil (100) pour influencer et/ou détecter des particules magnétiques dans un champ de vision (28), lequel l'appareil comprend :
- des moyens de sélection comprenant une unité génératrice de signal de champs de sélection (110) et des éléments de champs de sélection (116) pour générer un champ de sélection magnétique (50) ayant un motif dans l'espace de sa force de champ magnétique de sorte qu'une première sous-zone (52) ayant une faible force de champ magnétique où la magnétisation des particules magnétiques n'est pas saturée et qu'une seconde sous-zone (54) ayant une force magnétique supérieure où la magnétisation des particules magnétiques est saturée soient formées dans le champ de vision (28),
- des moyens d'excitation (120) comprenant une unité génératrice de signal de champs d'excitation (122) et des bobines de champs d'excitation (124 ; 125, 126, 127) pour changer la position dans l'espace des deux sous-zones (52, 54) dans le champ de vision (28) au moyen du champ d'excitation magnétique de sorte que la magnétisation du matériau magnétique change localement, et
- un ou plusieurs appareils à bobines (1, 2, 3, 4) selon la revendication 12 formant lesdites bobines de champs d'excitation, dans lequel l'unité génératrice de signal de champs d'excitation est couplée aux bornes CA des uns ou plusieurs agencements de câblage des uns ou plusieurs appareils à bobines.

14. Appareil selon la revendication 13,
comprenant en outre des moyens d'excitation et de réception comprenant ladite unité génératrice de signal de champs d'excitation (122), une unité de réception de signal (140) et une bobine de réception d'excitation (124), ladite bobine de réception d'excitation étant configurée pour changer la position dans l'espace des deux sous-zones (52, 54) dans le champ de vision (28) au moyen du champ d'excitation magnétique de sorte que la magnétisation du matériau magnétique change localement et pour acquérir des signaux de détection, lesquels signaux de détection dépendent de la magnétisation du champ de vision (28), laquelle magnétisation est influencée par le changement de position dans l'espace des première et seconde sous-zones (52, 54), dans lequel lesdits uns ou plusieurs appareils à bobines forment ladite bobine de réception d'excitation.

15. Procédé pour influencer et/ou détecter des particules magnétiques dans un champ de vision (28), lequel procédé comprend :
- la génération d'un champ de sélection magnétique (50) ayant un motif dans l'espace de sa force de champ magnétique de sorte qu'une première sous-zone (52) ayant une faible force de champ magnétique où la magnétisation des particules magnétiques n'est pas saturée et qu'une seconde sous-zone (54) ayant une force de champ magnétique supérieure où la magnétisation des particules magnétiques est saturée soient formées dans le champ de vision (28),
- le changement de position dans l'espace des deux sous-zones (52, 54) dans le champ de vision (28) au moyen d'un champ d'excitation magnétique de sorte que la magnétisation du matériau magnétique change localement, et
- la formation desdites bobines de champs d'excitation par un ou plusieurs appareils à bobines (1, 2, 3, 4) selon la revendication 12,
- l'application de courants CC aux bobines de champs d'excitation formées par un ou plusieurs agencements de câblage (1000, 2000, 3000, 4000) selon la revendication 1 et utilisées pour générer le champ d'excitation magnétique, lesdits courants CC étant configurés pour engendrer des contaminations magnétiques des premiers et seconds conducteurs des uns ou plusieurs agencements de câblage à saturation et pour ne pas générer de champ magnétique statique supplémentaire ou seulement dans la première sous-zone (52).
